# EUROPEAN PATENT APPLICATION

(11) **EP 4 238 963 A1**
(43) Date of publication of application: **06.09.2023**
(21) Application number: 21886807.3
(22) Date of filing: 27.10.2021
(51) Int. Cl.: C07D 211/14, A61K 31/451, A61P 25/28

(54) **ACID ADDITION SALT OF INDENE DERIVATIVE PRODRUG AND METHOD FOR PREPARING SAME**

(30) Priority: 02.11.2020 KR 20200144721
(71) Applicant: Chong Kun Dang Pharmaceutical Corp., Seoul 03742 (KR)
(72) Inventor: KIM, Jaeeun, Yongin-si Gyeonggi-do 16995 (KR); LEE, Jaemin, Yongin-si Gyeonggi-do 16995 (KR); NAM, Dong Hyuk, Yongin-si Gyeonggi-do 16995 (KR); PARK, So Hyun, Yongin-si Gyeonggi-do 16995 (KR); KANG, Sung Kwon, Yongin-si Gyeonggi-do 16995 (KR); PARK, Shin Jung, Yongin-si Gyeonggi-do 16995 (KR)
(74) Representative: J A Kemp LLP
(86) International application number: PCT/KR2021/015236
(87) International publication number: WO 2022/092813

(57) **Abstract**

The present invention relates to donepezil ether palmitate hydrochloride (DEP HCl) and a method for preparing same. Donepezil ether palmitate hydrochloride (DEP HCl) has been developed to have increased stability by suppressing generation of unknown related substances generated during a preparation process of a raw material and formulation of donepezil ether palmitate (DEP). In addition, the present invention relates to a sustained-release pharmaceutical composition comprising donepezil ether palmitate hydrochloride (DEP HCl) as a main ingredient. When administered in the body, the initial release of donepezil, which is an active ingredient, is reduced, thereby reducing the risk of side effects such as drug toxicity, and the constant release of donepezil in the body for a long period of time can improve the drug treatment effect on dementia patients.

## Description

### [Technical Field]

The present disclosure relates to an acid addition salt of a donepezil prodrug and a method for preparing the same. Specifically, the present disclosure relates to an acid addition salt of donepezil ether palmitate (DEP) and a method for preparing the same, and more specifically, to 2-((1-benzylpiperidin-4-yl)methyl)-5,6-dimethoxy-1H-inden-3-yl 2-(tetradecyloxy)acetate hydrochloride having excellent storage stability compared to conventional free bases, a pharmaceutical composition comprising the same, and a method for preparing the same.

### [Background Art]

Dementia is a syndrome that refers to a condition causing difficulty in daily life due to the decline in cognitive function caused by various diseases in the brain, and has major symptoms such as deterioration in language ability, comprehension ability, judgment ability, and personality change. Alzheimer's disease is the most common degenerative brain disease that causes senile dementia, and the fundamental treatment of Alzheimer's disease has not yet been fully developed, but various drugs capable of alleviating the symptoms of the disease and delaying the progression are being used clinically.

It is known that the neurotransmitter, acetylcholine (ACh) is less in the brain of dementia patients compared to normal people. Thus, research is being conducted in the direction of increasing the amount of acetylcholine in the brain or increasing the activity of cholinergic neurons.

A representative drug is acetylcholine degradation enzyme inhibitor. Acetylcholinesterase (AChE), an enzyme that degrades acetylcholine, is an enzyme that hydrolyzes acetylcholine into choline and acetate, and acetylcholinesterase (AChE) inhibitors that inhibit this degradation enzyme are widely used as a treatment for Alzheimer's disease. Representative acetylcholinesterase (AChE) inhibitors include Donepezil (brand name: Aricept), and the most common dosage form is an oral dosage form. However, it is known in some patients that oral administration of donepezil tablets causes gastrointestinal side effects such as diarrhea, nausea, loss of appetite, muscle convulsions, and the like. The oral preparations of donepezil hydrochloride that are currently commercially available are generally administered at a starting dose of 5 mg once a day at bedtime and maintained for 4 to 6 weeks, and then the dose is increased to 10 mg once a day. Therefore, patients have very poor medication compliance because the preparations have to be administered orally every day. In order to improve the poor medication compliance, oral disintegrating tablets are commercially available, but they also have a problem in that active ingredients must be continuously administered over a long period of time.

In order to overcome these problems, donepezil ether palmitate represented by the following Chemical Formula 1 was developed to maintain the concentration of the drug continuously for a long time, thereby reducing the frequency of drug administration and ultimately improving the patient's convenience and compliance:

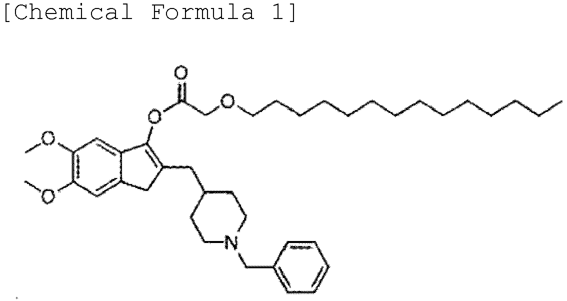

However, it was confirmed that an unknown related substance in addition to known related substances was greatly increased when conducting the study of the raw material and formulation of donepezil ether palmitate represented by Chemical Formula 1 above. As a result of structural analysis after separating the unknown related substance, a 2-((1-benzylpiperidin-4-yl)methyl)-5,6-dimethoxy-1*H*-inden-1-yl 2-(tetradecyloxy)acetate (PT impurity) compound represented by the following Chemical Formula 2 was found:

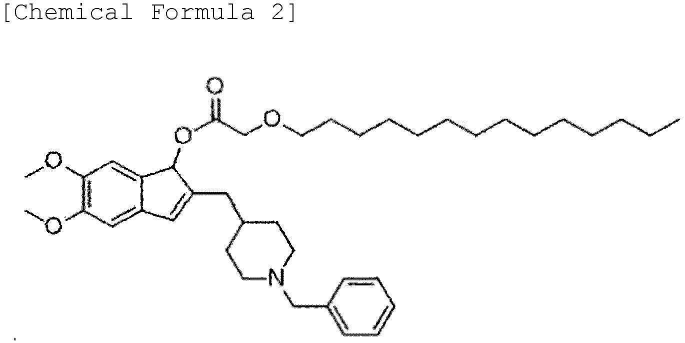

In this regard, it has been reported that intramolecular rearrangement may occur due to the transfer of hydrogen (1,3-proton transfer) in the skeleton of an indene structure as shown in Reaction Scheme 1 below (Acta Chemica Scandinavia, 1998, 52, 541-548).

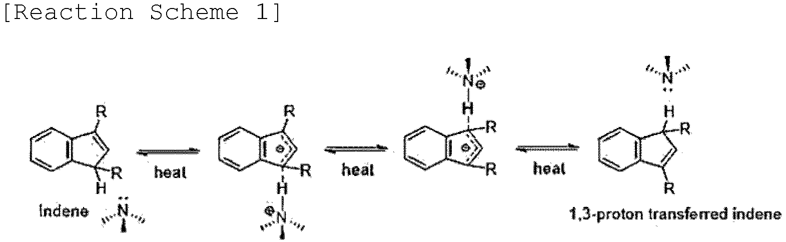

This intramolecular rearrangement is caused by a complex action of three elements: an indene skeleton, an amine base, and heat. This may also be applied to donepezil ether palmitate containing an indene skeleton. Since donepezil ether palmitate contains an indene skeleton and an amine functional group in a structure thereof, when heat is applied, the PT impurity represented by Chemical Formula 2 may be produced as shown in Reaction Scheme 2 below:

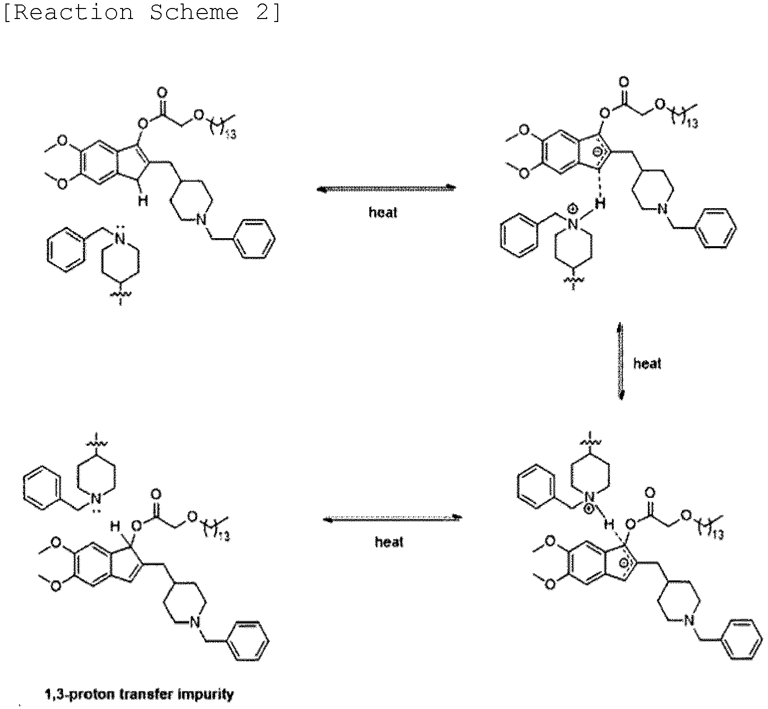

As described above, the present inventors confirmed that the indene skeleton, amine, and heat were the causes of PT impurity, and made an effort to develop a donepezil prodrug with increased stability by suppressing the generation of PT impurity through the exclusion of these factors.

### [Related Art Documents]

### [Patent Documents]

(Patent Document 1) Japanese Laid-Open Patent Publication (Hei) No. 11-315016
(Patent Document 2) Korea Patent Registration No. 1980534

### [Non-Patent Document]

(Non-Patent Document 1) Acta Chemica Scandinavia, 1998, 52, 541-548

### [Disclosure]

### [Technical Problem]

In order to suppress production of the PT impurity, it is required to remove at least one of three factors of the indene skeleton, amine, and heat. However, since it is difficult to remove the indene structure, which is the basic skeleton, and the thermal factor, the present inventors have attempted to suppress the generation of PT impurity by blocking the amine functional group.

Accordingly, an object of the present disclosure is to provide an acid addition salt of donepezil ether palmitate with improved stability by introducing an acid addition salt into donepezil ether palmitate to block the base action of amine.

In addition, another object of the present disclosure is to provide an acid addition salt of donepezil ether palmitate capable of reducing the initial release of donepezil to lower the risk of side effects such as drug toxicity, and allowing the constant release of donepezil in the body for a long period of time, thereby improving the drug treatment effect of patients with dementia, and a sustained-release pharmaceutical composition comprising the same.

### [Technical Solution]

In order to achieve the above object, in one general aspect, the present disclosure provides an acid addition salt of a compound represented by the following Chemical Formula 3:

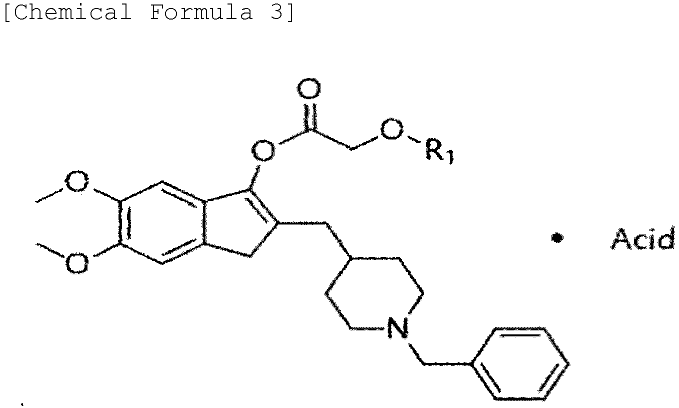

in Chemical Formula above, R₁ may be C₁₂-C₁₆ alkyl, and
the acid may be hydrochloric acid, sulfuric acid, phosphoric acid, nitric acid, carbonic acid, citric acid, acetic acid, formic acid, fumaric acid, glucaric acid, gluconic acid, glutamic acid, ascorbic acid, benzoic acid, camphorsulfonic acid, capric acid, caproic acid, caprylic acid, cinnamic acid, lauric acid, maleic acid, malic acid, malonic acid, mandelic acid, oleic acid, orotic acid, benzenesulfonic acid, tartaric acid, vanillic acid, napadisilic acid, tosylic acid, mesylic acid, succinic acid, ethanesulfonic acid, lactic acid, butyric acid, propionic acid, nicotinic acid, or oxalic acid, but is not limited thereto.

In another general aspect, the present disclosure provides a sustained-release pharmaceutical composition for preventing or treating dementia comprising the compound represented by Chemical Formula 3 above.

The present disclosure provides donepezil ether palmitate hydrochloride (DEP HCl) represented by the following Chemical Formula 4:

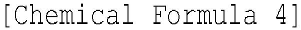

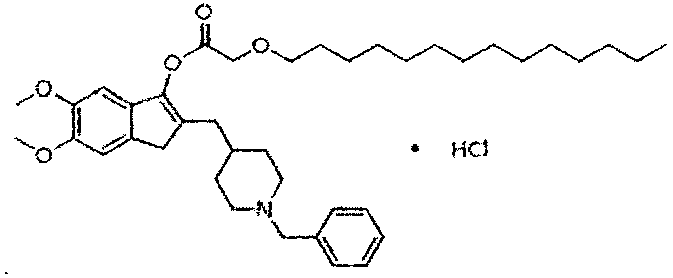

In still another general aspect, the present disclosure provides a sustained-release pharmaceutical composition for preventing or treating dementia comprising the donepezil ether palmitate hydrochloride (DEP HCl) compound represented by Chemical Formula 4 above.

The chemical name of the compound represented by Chemical Formula 4 is 2-((1-benzylpiperidin-4-yl)methyl)-5,6-dimethoxy-1*H*-inden-3-yl 2-(tetradecyloxy)acetate hydrochloride, and referred to herein as donepezil ether palmitate hydrochloride.

The donepezil ether palmitate hydrochloride of the present disclosure may be prepared by reacting donepezil ether palmitate with hydrochloric acid.

The donepezil ether palmitate may be prepared through the following steps:
(1) mixing 1-tetradecanol, sodium chloroacetate, and potassium hydroxide to obtain sodium 2-(tetradecyloxy)acetate;
(2) reacting the sodium 2-(tetradecyloxy)acetate with an aqueous HCl solution to obtain 2-(tetradecyloxy)acetic acid;
(3) reacting the 2-(tetradecyloxy)acetic acid with oxalyl chloride to obtain 2-(tetradecyloxy)acetyl chloride; and
(4) reacting the 2-(tetradecyloxy)acetyl chloride with donepezil free base.

The donepezil ether palmitate hydrochloride of the present disclosure may be prepared through the following steps:
(1) preparing a solution by adding and dissolving a solvent into
2-((1-benzylpiperidin-4-yl)methyl)-5,6-dimethoxy-1*H-*inden-3-yl 2-(tetradecyloxy)acetate;
(2) forming a salt by adding an acid to the solution prepared in step (1); and
(3) obtaining a solid by stirring the salt prepared in step (2).

### [Advantageous Effects]

The present disclosure provides donepezil ether palmitate hydrochloride capable of suppressing the production of PT impurity, which is an unknown related substance of donepezil ether palmitate, and an efficient preparation method thereof.

The donepezil ether palmitate hydrochloride may not only suppress production of PT impurity, but also increase the melting point of raw materials to have excellent thermal stability and easy storage, thereby making it possible to be usefully employed as a treatment for symptoms of Alzheimer's dementia.

The donepezil ether palmitate hydrochloride of the present disclosure may exhibit a significantly superior release pattern than donepezil and have a low initial release to maintain a sustained-release for a long period of time without rapid drug release, thereby having an effect of improving the drug treatment compliance of patients with dementia.

### [Description of Drawings]

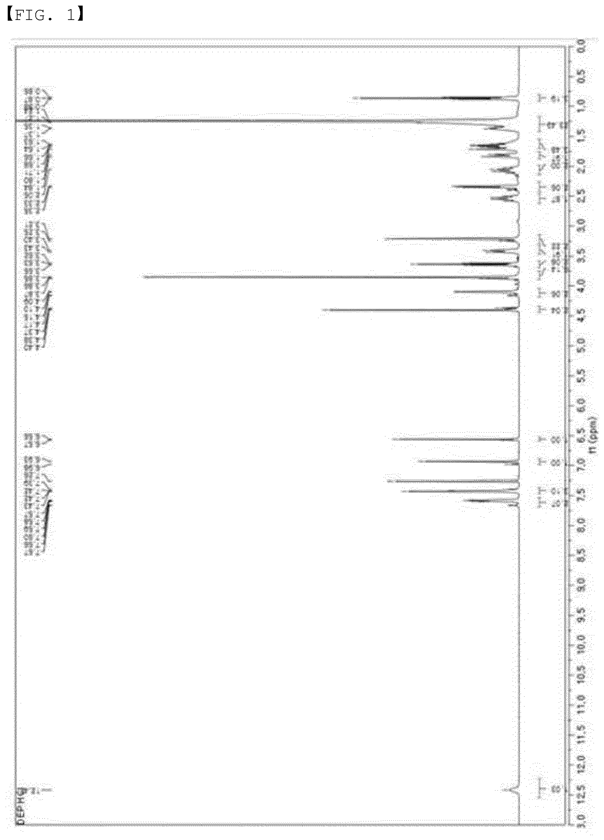

FIG. 1 shows results of nuclear magnetic resonance analysis (¹H NMR) of donepezil ether palmitate hydrochloride (DEP HCl) synthesized in Example 6.

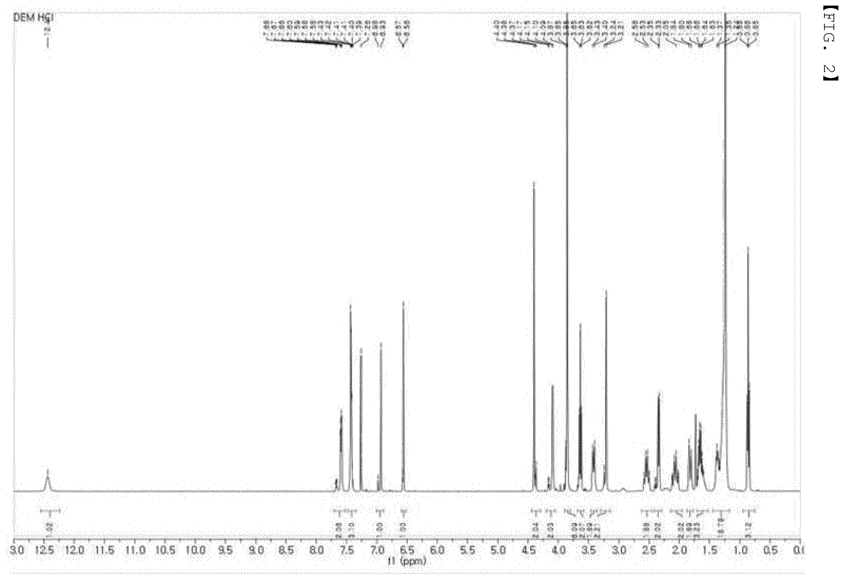

FIG. 2 shows results of nuclear magnetic resonance analysis (¹H NMR) of donepezil ether myristate hydrochloride (DEM HCl) synthesized in Example 7.

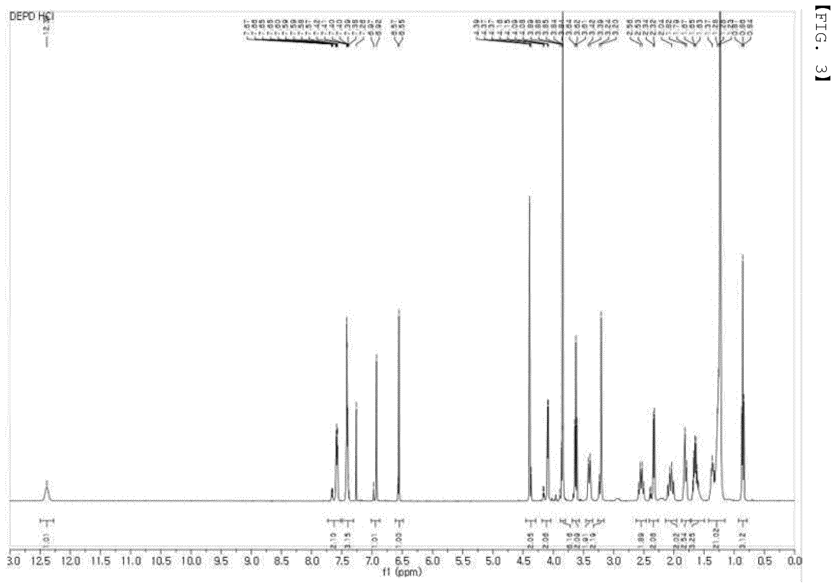

FIG. 3 shows results of nuclear magnetic resonance analysis (¹H NMR) of donepezil ether pentadecanoate hydrochloride (DEPD HCl) synthesized in Example 8.

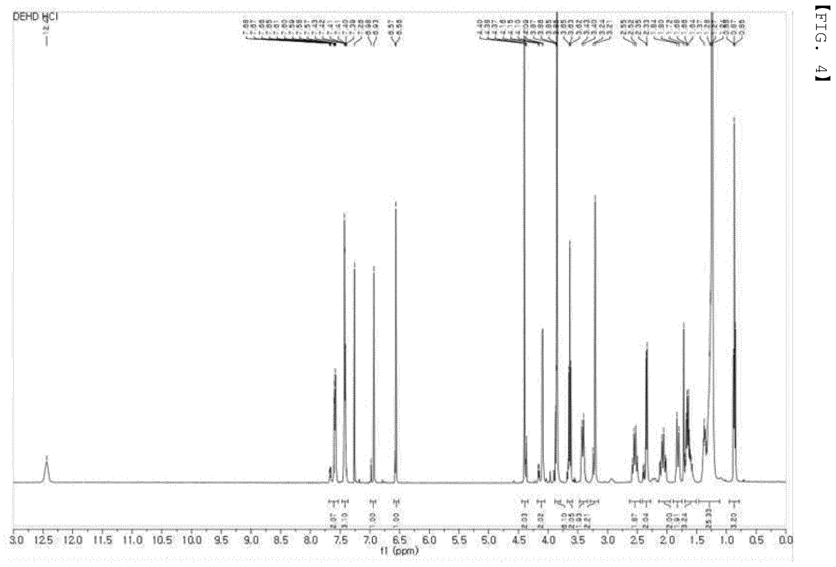

FIG. 4 shows results of nuclear magnetic resonance analysis (¹H NMR) of donepezil ether heptadecanoate hydrochloride (DEHD HCl) synthesized in Example 9.

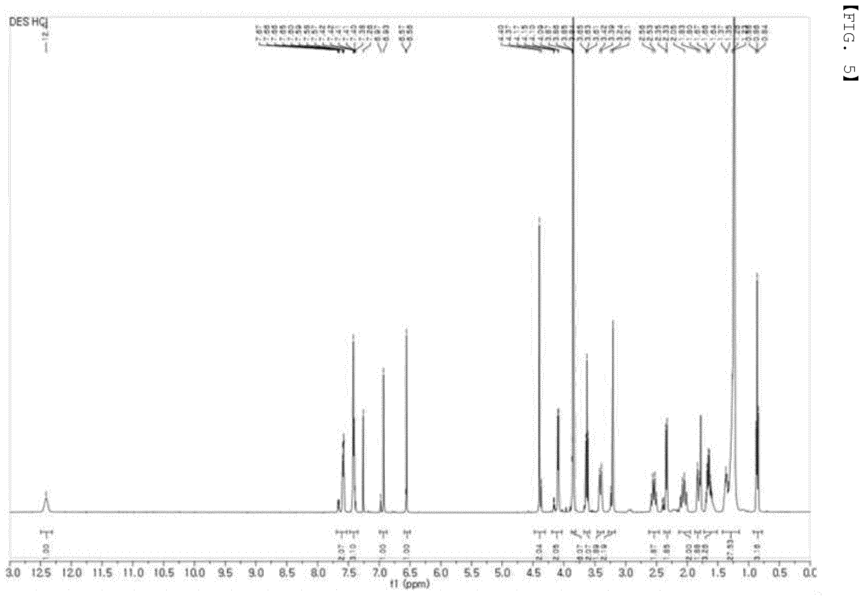

FIG. 5 shows results of nuclear magnetic resonance analysis (¹H NMR) of donepezil ether stearate hydrochloride (DES HCl) synthesized in Example 10.

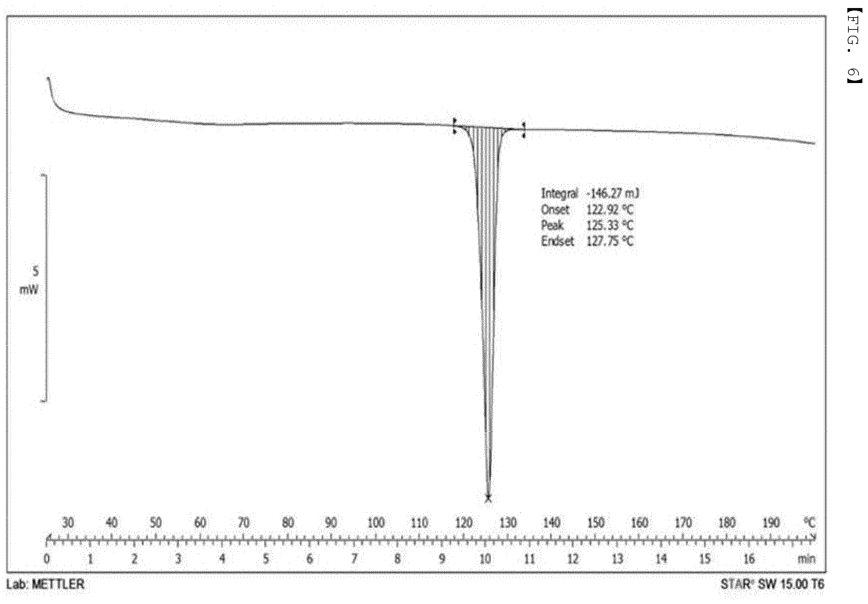

FIG. 6 shows differential scanning calorimetry (DSC) analysis data of donepezil ether palmitate hydrochloride (DEP HCl) synthesized in Example 6.

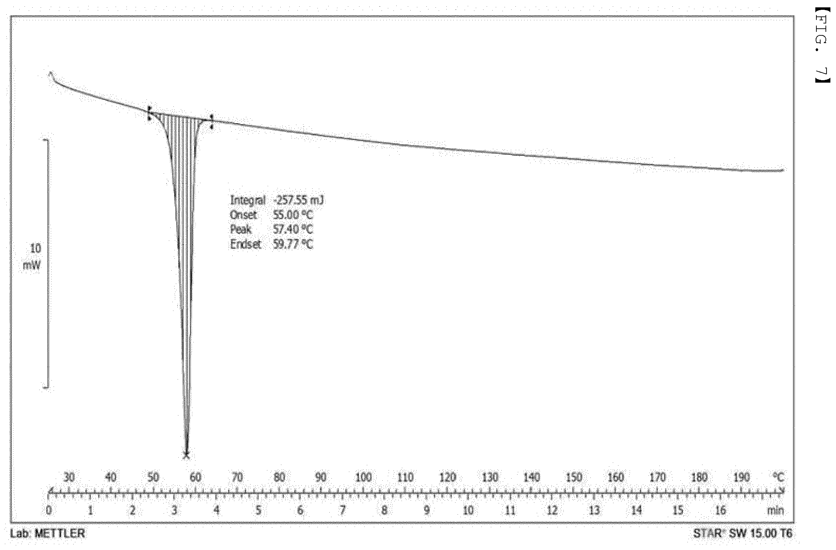

FIG. 7 shows differential scanning calorimetry (DSC) analysis data of donepezil ether palmitate (DEP) synthesized in Example 1.
FIG. 8 shows results of the concentration of donepezil (D) in blood after administration of donepezil (D) to rats.
FIG. 9 shows results of the concentration of donepezil in blood after administration of donepezil ether palmitate hydrochloride (DEP HCl) to rats.

### [Best Mode]

Hereinafter, preferred embodiments are provided to aid understanding of the present disclosure. However, the following Examples are only provided to understand the present disclosure more easily, but the content of the present disclosure is not limited by these Examples.

In the present disclosure, donepezil has a structure of Chemical Formula 9 below, and has a chemical name of 2-((1-benzylpiperidin-4-yl)methyl)-5,6-dimethoxy-2,3-hydro-1H-inden-1-one. The donepezil free base of the present disclosure may be directly prepared by a known method or purchased.

The present disclosure relates to an acid addition salt of a compound represented by Chemical Formula 3 below: 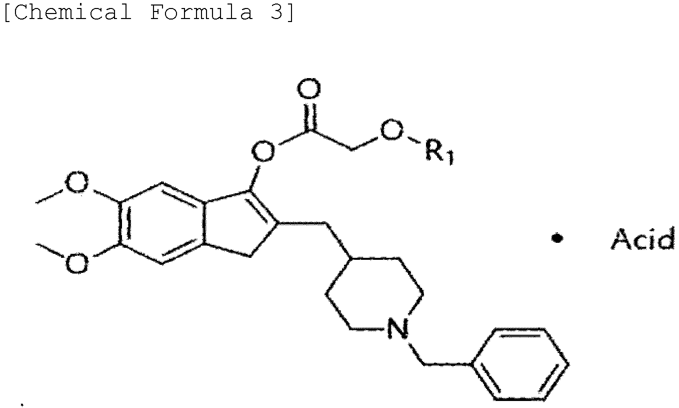
in Chemical Formula above, R₁ is C₁₂-C₁₆ alkyl, and
the acid is hydrochloric acid, sulfuric acid, phosphoric acid, nitric acid, carbonic acid, citric acid, acetic acid, formic acid, fumaric acid, glucaric acid, gluconic acid, glutamic acid, ascorbic acid, benzoic acid, camphorsulfonic acid, capric acid, caproic acid, caprylic acid, cinnamic acid, lauric acid, maleic acid, malic acid, malonic acid, mandelic acid, oleic acid, orotic acid, benzenesulfonic acid, tartaric acid, vanillic acid, napadisilic acid, tosylic acid, mesylic acid, succinic acid, ethanesulfonic acid, lactic acid, butyric acid, propionic acid, nicotinic acid, or oxalic acid.

The present disclosure relates to donepezil ether palmitate hydrochloride (DEP HCl) represented by Chemical Formula 4 below:

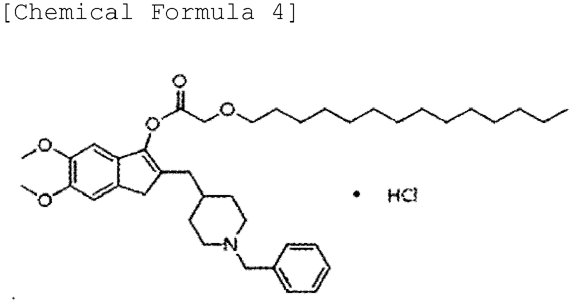

The present disclosure relates to donepezil ether myristate hydrochloride (DEM HCl) represented by Chemical Formula 5 below:

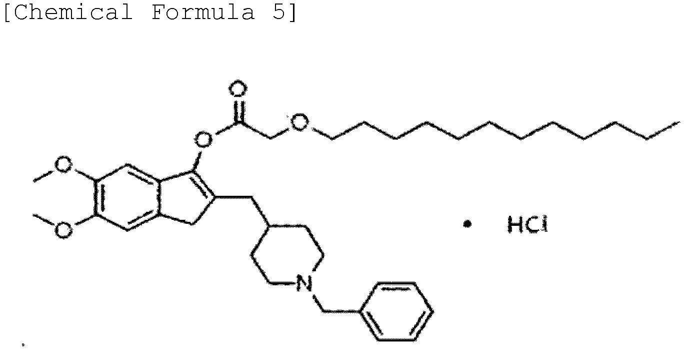

The present disclosure relates to donepezil ether pentadecanoate hydrochloride (DEPD HCl) represented by Chemical Formula 6 below:
[Chemical Formula 6]

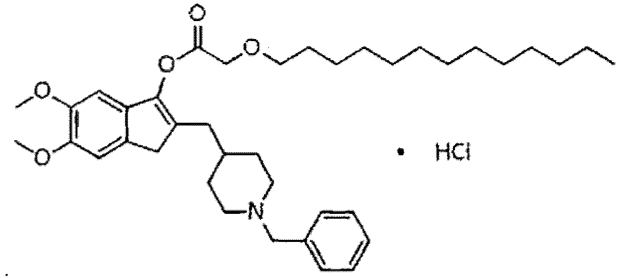

The present disclosure relates to donepezil ether heptadecanoate (DEHD HCl) represented by Chemical Formula 7 below:

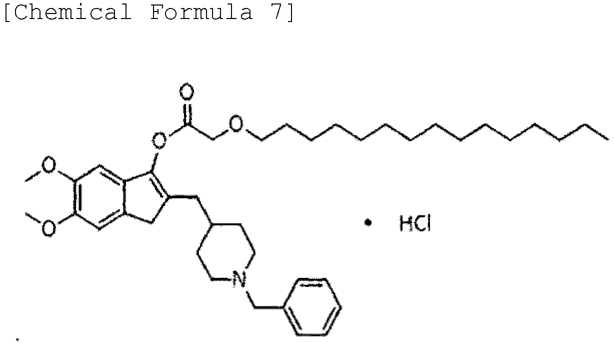

The present disclosure relates to donepezil ether stearate hydrochloride (DES HCl) represented by Chemical Formula 8 below:

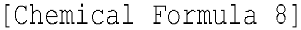

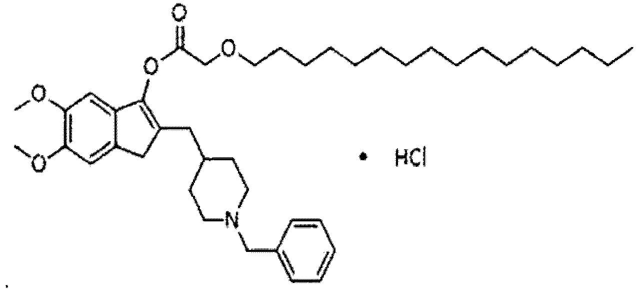

The donepezil free base of the present disclosure may be directly prepared by a known method or purchased.

Various reagents and solvents used in Examples of the present disclosure were purchased from Sigma-Aldrich Korea Ltd., TCI Co., Ltd., and Daejung Chemicals and Metals Co., Ltd., and the like. The structure of the donepezil ether palmitate hydrochloride of the present disclosure was confirmed through nuclear magnetic resonance analysis (¹H NMR) and hydrochloride titration (potentiometric titration). Further, it was confirmed through differential scanning calorimetry (DSC) and melting point analysis that donepezil ether palmitate and hydrochloride salts thereof had differences in physicochemical properties.

In the present disclosure, HPLC was measured using a 1260 infinity LC manufactured by Agilent Technologies, and ¹H NMR was measured using a 400 ultrashield NMR Spectrometer manufactured by Bruker. The purity was measured as area (%) by HPLC.

The HPLC conditions used in the present disclosure were as follows, and the purity of donepezil ether palmitate hydrochloride was measured.
1) detector: ultraviolet absorption photometer (measurement wavelength: 276 nm)
2) column : YMC-Pack Pro C18(4.6 × 250 mm, 5 um)
3) mobile phase A: Solution obtained by dissolving 1.88 g of sodium 1-hexanesulfonate in 1000 mL of purified water, and then adding 1.0 mL of phosphoric acid thereto, wherein each amount of reaction substances was accurately weighed.
4) mobile phase B: acetonitrile

| Time | Mobile phase A | Mobile phase B |
|---|---|---|
| 0 | 60 | 40 |
| 10 | 30 | 70 |
| 30 | 30 | 70 |
| 31 | 20 | 80 |
| 35 | 20 | 80 |
| 40 | 60 | 40 |
| 50 | 60 | 40 |

5) flow rate: 1.0 mL / min
6) sample: donepezil ether palmitate hydrochloride 25 mg / mobile phase 10 mL
7) injection volume: 5 *µ*ℓ
8) column temperature: constant temperature around 50 °C

### [Example 1]

### Preparation of donepezil ether palmitate (DEP) [2-((1-benzylpiperidin-4-yl)methyl)-5,6-dimethoxy-1H-inden-3-yl 2-(tetradecyloxy)acetate]

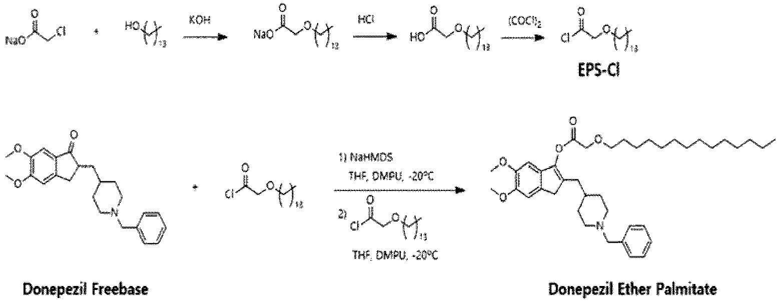

### 1) Preparation of sodium 2-(tetradecyloxy) acetate (EPS-Na)

To the reaction vessel, 400 g of 1-tetradecanol was added and dissolved at 60 °C, and then 108.7 g of sodium chloroacetate and 108 mL of n-heptane were added. To the reaction solution, 78.5 g of potassium hydroxide was added, and the mixture was heated to 85 °C and stirred for 3 hours. Into the reaction solution, 980 mL of n-heptane and 2.2 L of ethanol (EtOH) were injected, stirred at 60 °C for 1 hour, and cooled to room temperature, followed by filtration to obtain 263.3 g of sodium 2-(tetradecyloxy)acetate compound.

(¹H NMR (CDCl₃, 400 MHz) δ 3.83 (s, 2H), 3.48 (t, J = 6.9 Hz, 2H), 1.65 - 1.57 (m, 2H), 1.37 - 1.29 (m, 22H), 0.90 (t, J = 6.9 Hz, 3H)).

### 2) Preparation of 2-(tetradecyloxy)acetic acid (EPS-acid)

To the reaction vessel, 263.3 g of sodium 2-(tetradecyloxy)acetate prepared in 1) above was added to 2.2 L of ethyl acetate (EtOAc) and stirred, and then 1.45 L of 2M HCl aqueous solution was injected and stirred at room temperature for 2 hours. The resulting product was subjected to layer separation to obtain an organic layer, and washed twice with 1.45 L of purified water. The obtained organic layer was washed with 1.45 L of saturated aqueous sodium chloride solution. After the organic layer was obtained, dried over anhydrous sodium sulfate, filtered, and concentrated under reduced pressure. The concentrated compound was dissolved in 1.63 L of n-hexane, and then the mixture was stirred at 0 °C for 1 hour. The precipitated solid was filtered to obtain 110.6 g of a 2-(tetradecyloxy)acetic acid compound.

(¹H NMR (CDCl₃, 400 MHz) δ 4.11 (s, 2H), 3.56 (t, J = 6.7 Hz, 2H), 1.66 - 1.59 (m, 2H), 1.36 - 1.25 (m, 22H), 0.88 (t, *J* = 7.2 Hz, 3H)).

### 3) Preparation of 2-(tetradecyloxy)acetyl chloride (EPS-Cl)

To the reaction vessel, 50.0 g of 2-(tetradecyloxy)acetic acid prepared in 2) above was added and dissolved by injecting 500 mL of dichloromethane and 0.5 mL of dimethylformamide. Into the reaction solution, 16.5 mL of oxalyl chloride was injected and stirred at room temperature for 3 hours. After the reaction was completed, the solvent was removed by distillation under reduced pressure to obtain 53.4 g of 2-(tetradecyloxy)acetyl chloride compound.
(¹H NMR (CDCl₃, 400 MHz) δ 4.39 (s, 2H), 3.56 (t, *J* = 6.6 Hz, 2H), 1.64 ∼ 1.57 (m, 2H), 1.36 ∼ 1.25 (m, 22H), 0.88 (t, *J* = 6.8 Hz, 3H))

### 4) Preparation of 2-((1-benzylpiperidin-4-yl)methyl)-5,6-dimethoxy-1H-inden-3-yl 2-(tetradecyloxy)acetate (DEP)

To the reaction vessel, 63.3 g of donepezil free base (purchased from "Jinan Chenghui-Shuangda Chemical Co. Ltd") was added, and then 190 mL of THF (tetrahydrofuran) and 127 mL of DMPU (1,3-dimethyl-3,4,5,6-tetrahydro-2(1H)-pyrimidinone) were injected, dissolved, and cooled to -20 °C. Into the reaction solution, 183.5 mL of NaHMDS (1.0M THF solution) was injected for 30 minutes, and the mixture was stirred at -20 °C for 1 hour. The reaction solution was injected for 40 minutes into a solution in which 53.4 g of 2-(tetradecyloxy)acetyl chloride prepared in 'Example 1-3)' was dissolved in 380 mL of THF (tetrahydrofuran) and 253 mL of DMPU (1,3-dimethyl-3,4,5,6-tetrahydro-2(1*H*)-pyrimidinone) at -20 °C, and stirred for 1 hour at the same temperature.

Into the reaction solution, 630 mL of saturated aqueous ammonium chloride solution was injected, filtered through celite, and then washed with 630 mL of ethyl acetate (EtOAc). The resulting product was subjected to layer separation to obtain an organic layer, and the aqueous layer was backextracted with 630 mL of ethyl acetate (EtOAc). The combined organic layer was washed three times with 630 mL of 5% aqueous sodium chloride solution. The obtained organic layer was dried over anhydrous sodium sulfate, filtered, and concentrated under reduced pressure. Into the concentrated solution, 630 mL of n-hexane was injected and stirred at 40 °C, and the temperature was lowered back to room temperature and the slurry was stirred for 1 hour. The solution was filtered and the resulting filtrate was concentrated under reduced pressure. The concentrated solution was purified with a silica column and recrystallized with 630 mL of n-heptane to obtain 33.1 g of donepezil ether palmitate (DEP).
(¹H NMR (CDCl₃, 400 MHz) δ 7.32 ~ 7.21 (m, 5H), 6.96 (s, 1H), 6.59 (s, 1H), 4.37 (s, 2H), 3.88 (s, 3H), 3.87 (s, 3H), 3.64 (t, *J* = 6.7 Hz, 2H), 3.47 (s, 2H), 3.25 (s, 2H), 2.87 ∼ 2.84 (m, 2H), 2.27 (d, *J* = 7.1 Hz, 2H), 1.90 (t, *J* = 10.7 Hz, 2H), 1.71 ∼ 1.63 (m, 4H), 1.55 ∼ 1.44 (m, 1H), 1.41 ∼ 1.25 (m, 24H), 0.88 (t, *J* = 6.8 Hz, 3H))

### [Example 2]

### Preparation of donepezil ether myristate (DEM) [2-((1-benzylpiperidin-4-yl)methyl)-5,6-dimethoxy-1H-inden-3-yl 2-(dodecyloxy)acetate]

### 1) Preparation of sodium 2-(dodecyloxy)acetate (EMS-Na)

It was prepared in a similar manner to 1) of Example 1 using 1-dodecanol.

(¹H NMR (CD₃OD, 400 MHz) δ 3.83 (s, 2H), 3.47 (t, *J* = 6.9 Hz, 2H), 1.64 ∼ 1.57 (m, 2H), 1.37 ∼ 1.29 (m, 18H), 0.90 (t, *J* = 6.9 Hz, 3H)).

### 2) Preparation of 2-(dodecyloxy)acetic acid (EMS-acid)

It was prepared in a similar manner to 2) of Example 1 using the EMS-Na prepared in 1) above.

(¹H NMR (CDCl₃, 400 MHz) δ 4.11 (s, 2H), 3.56 (t, *J* = 6.7 Hz, 2H), 1.66 ∼ 1.59 (m, 2H), 1.36 ∼ 1.25 (m, 18H), 0.87 (t, *J* = 6.9 Hz, 3H)).

### 3) Preparation of 2-(dodecyloxy)acetyl chloride (EMS-Cl)

It was prepared in a similar manner to 3) of Example 1 using the EMS-acid prepared in 2) above.

(¹H NMR (CDCl₃, 400 MHz) δ 4.39 (s, 2H), 3.57 (t, *J* = 6.6 Hz, 2H), 1.64 ∼ 1.57 (m, 2H), 1.36 ∼ 1.26 (m, 18H), 0.88 (t, *J* = 6.8 Hz, 3H)).

### 4) Preparation of 2-((1-benzylpiperidin-4-yl)methyl)-5,6-dimethoxy-1H-inden-3-yl 2-(dodecyloxy)acetate (DEM)

It was prepared in a similar manner to 4) of Example 1 using the EMS-Cl prepared in 3) above.

(¹H NMR (CDCl₃, 400 MHz) δ 7.32 ~ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ∼ 2.84 (m, 2H), 2.27 (d, *J* = 7.1 Hz, 2H), 1.91 (t, *J* = 10.8 Hz, 2H), 1.71 - 1.63 (m, 4H), 1.55 - 1.44 (m, 1H), 1.41 - 1.25 (m, 20H), 0.88 (t, *J* = 6.8 Hz, 3H)).

### [Example 3]

### Preparation of donepezil ether pentadecanoate (DEPD) [2-((1-benzylpiperidin-4-yl)methyl)-5,6-dimethoxy-1H-inden-3-yl 2-(tridecyloxy)acetate]

### 1) Preparation of sodium 2-(tridecyloxy)acetate (EPDS-Na)

It was prepared in a similar manner to 1) of Example 1 using 1-tridecanol.

(¹H NMR (CD₃OD, 400 MHz) δ 3.83 (s, 2H), 3.47 (t, *J* = 6.9 Hz, 2H), 1.64 ∼ 1.57 (m, 2H), 1.37 ∼ 1.29 (m, 20H), 0.90 (t, *J* = 6.8 Hz, 3H)).

### 2) Preparation of 2-(tridecyloxy)acetic acid (EPDS-acid)

It was prepared in a similar manner to 2) of Example 1 using the EPDS-Na prepared in 1) above.

(¹H NMR (CDCl₃, 400 MHz) δ 4.10 (s, 2H), 3.56 (t, *J* = 6.7 Hz, 2H), 1.66 ∼ 1.59 (m, 2H), 1.37 ∼ 1.26 (m, 20H), 0.88 (t, *J* = 6.8 Hz, 3H)).

### 3) Preparation of 2-(tridecyloxy)acetyl chloride (EPDS-Cl)

It was prepared in a similar manner to 3) of Example 1 using the EPDS-acid prepared in 2) above.

(¹H NMR (CDCl₃, 400 MHz) δ 4.39 (s, 2H), 3.57 (t, *J* = 6.6 Hz, 2H), 1.64 ∼ 1.57 (m, 2H), 1.36 ∼ 1.26 (m, 20H), 0.88 (t, *J* = 6.8 Hz, 3H)).

### 4) Preparation of 2-((1-benzylpiperidin-4-yl)methyl)-5,6-dimethoxy-1H-inden-3-yl 2-(tridecyloxy)acetate (DEPD)

It was prepared in a similar manner to 4) of Example 1 using the EPDS-Cl prepared in 3) above.

(¹H NMR (CDCl₃, 400 MHz) δ 7.32 ~ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ∼ 2.84 (m, 2H), 2.27 (d, *J* = 7.1 Hz, 2H), 1.91 (t, *J* = 10.8 Hz, 2H), 1.71 - 1.63 (m, 4H), 1.55 ∼ 1.44 (m, 1H), 1.43 ∼ 1.25 (m, 22H), 0.88 (t, *J* = 6.8 Hz, 3H)).

### [Example 4]

### Preparation of donepezil ether heptadecanoate (DEHD) [2-((1-benzylpiperidin-4-yl)methyl)-5,6-dimethoxy-1H-inden-3-yl 2-(pentadecyloxy)acetate]

### 1) Preparation of sodium 2-(pentadecyloxy)acetate (EHDS-Na)

It was prepared in a similar manner to 1) of Example 1 using 1-pentadecanol.

(¹H NMR (CD₃OD, 400 MHz) δ 3.83 (s, 2H), 3.47 (t, *J* = 6.9 Hz, 2H), 1.64 ∼ 1.57 (m, 2H), 1.37 ∼ 1.29 (m, 24H), 0.90 (t, *J* = 6.9 Hz, 3H)).

### 2) Preparation of 2-(pentadecyloxy)acetic acid (EHDS-acid)

It was prepared in a similar manner to 2) of Example 1 using the EHDS-Na prepared in 1) above.

(¹H NMR (CDCl₃, 400 MHz) δ 4.11 (s, 2H), 3.56 (t, *J* = 6.7 Hz, 2H), 1.69 ∼ 1.56 (m, 2H), 1.37 ∼ 1.25 (m, 24H), 0.88 (t, *J* = 6.8 Hz, 3H)).

### 3) Preparation of 2-(pentadecyloxy)acetyl chloride (EHDS-Cl)

It was prepared in a similar manner to 3) of Example 1 using the EHDS-acid prepared in 2) above.

(¹H NMR (CDCl₃, 400 MHz) δ 4.39 (s, 2H), 3.57 (t, J = 6.6 Hz, 2H), 1.64 - 1.57 (m, 2H), 1.39 - 1.25 (m, 24H), 0.88 (t, *J* = 6.8 Hz, 3H)).

### 4) Preparation of 2-((1-benzylpiperidin-4-yl)methyl)-5,6-dimethoxy-1H-inden-3-yl 2-(pentadecyloxy)acetate (DEHD)

It was prepared in a similar manner to 4) of Example 1 using the EHDS-Cl prepared in 3) above.

(¹H NMR (CDCl₃, 400 MHz) δ 7.32 ∼ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ∼ 2.84 (m, 2H), 2.27 (d, *J* = 7.1 Hz, 2H), 1.91 (t, *J* = 10.8 Hz, 2H), 1.71 ∼ 1.63 (m, 4H), 1.53 ∼ 1.45 (m, 1H), 1.41 ∼ 1.25 (m, 26H), 0.88 (t, *J* = 6.8 Hz, 3H)).

### [Example 5]

### Preparation of donepezil ether stearate (DES) [2-((1-benzylpiperidin-4-yl)methyl)-5,6-dimethoxy-1H-inden-3-yl 2-(hexadecyloxy)acetate]

### 1) Preparation of sodium 2-(hexadecyloxy)acetate (ESS-Na)

It was prepared in a similar manner to 1) of Example 1 using 1-hexadecanol.

(¹H NMR (CD₃OD, 400 MHz) δ 3.83 (s, 2H), 3.47 (t, *J* = 6.8 Hz, 2H), 1.64 ∼ 1.57 (m, 2H), 1.37 ∼ 1.29 (m, 26H), 0.90 (t, *J* = 6.8 Hz, 3H)).

### 2) Preparation of 2-(hexadecyloxy)acetic acid (ESS-acid)

It was prepared in a similar manner to 2) of Example 1 using the ESS-Na prepared in 1) above.

(¹H NMR (CDCl₃, 400 MHz) δ 4.10 (s, 2H), 3.57 (t, *J* = 6.7 Hz, 2H), 1.66 ∼ 1.59 (m, 2H), 1.37 ∼ 1.26 (m, 26H), 0.88 (t, *J* = 6.8 Hz, 3H)).

### 3) Preparation of 2-(hexadecyloxy)acetyl chloride (ESS-Cl)

It was prepared in a similar manner to 3) of Example 1 using the ESS-acid prepared in 2) above.

(¹H NMR (CDCl₃, 400 MHz) δ 4.39 (s, 2H), 3.56 (t, *J* = 6.6 Hz, 2H), 1.64 ∼ 1.57 (m, 2H), 1.36 ∼ 1.25 (m, 26H), 0.88 (t, *J* = 6.8 Hz, 3H)).

### 4) Preparation of 2-((1-benzylpiperidin-4-yl)methyl)-5,6-dimethoxy-1H-inden-3-yl 2-(hexadecyloxy)acetate (DES)

It was prepared in a similar manner to 4) of Example 1 using the ESS-Cl prepared in 3) above.

(¹H NMR (CDCl₃, 400 MHz) δ 7.32 ∼ 7.21 (m, 5H), 6.96 (s, 1H), 6.59 (s, 1H), 4.38 (s, 2H), 3.87 (s, 3H), 3.87 (s, 3H), 3.64 (t, *J* = 6.7 Hz, 2H), 3.47 (s, 2H), 3.25 (s, 2H), 2.87 ∼ 2.84 (m, 2H), 2.27 (d, *J* = 7.1 Hz, 2H), 1.91 (t, *J* = 10.9 Hz, 2H), 1.71 ∼ 1.63 (m, 4H), 1.52 - 1.45 (m, 1H), 1.41 ∼ 1.25 (m, 28H), 0.88 (t, *J* = 6.7 Hz, 3H)).

### [Example 6]

### Preparation of donepezil ether palmitate hydrochloride (DEP HCl) [2-((1-benzylpiperidin-4-yl)methyl)-5,6-dimethoxy-1H-inden-3-yl 2-(tetradecyloxy)acetate hydrochloride]

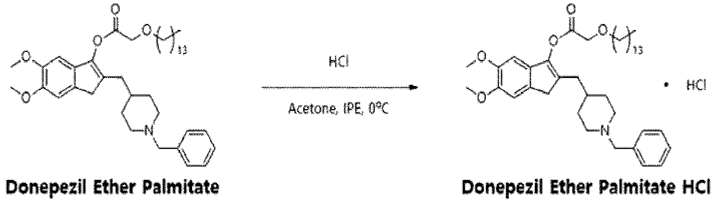

To the reaction vessel, donepezil ether palmitate (DEP) (5.0 g, 7.89 mmol) and 100 mL of acetone were added, heated up to 40 °C to dissolve, and then cooled to 10 °C again. Next, 1M HCl (7.73 mL, 7.73mmol) diluted in ethyl acetate (EtOAc) was added dropwise for 1 hour, and then the reaction mixture was cooled to 0 °C and stirred. After solid precipitation, the mixture was further stirred at 0 °C for 30 minutes, and 200 mL of isopropyl ether was added thereto, followed by stirring at the same temperature for 30 minutes. The precipitated solid was filtered and washed with 15 mL of cooled isopropyl ether to obtain 4.86 g of 2-((1-benzylpiperidin-4-yl)methyl)-5,6-dimethoxy-1*H*-inden-3-yl 2-(tetradecyloxy)acetate hydrochloride (DEP HCl).

(¹H NMR (CDCl₃, 400 MHz) δ 12.42 (br, 1H), 7.67 ∼ 7.57 (m, 2H), 7.43 ∼ 7.39 (m, 3H), 6.98 ∼ 6.93 (m, 1H), 6.57 ∼ 6.56 (m, 1H), 4.40 ∼ 4.37 (m, 2H), 4.17 ∼ 4.09 (m, 2H), 3.87 - 3.85 (m, 6H), 3.63 (t, *J* = 6.7 Hz, 2H), 3.43 ∼ 3.40 (m, 2H), 3.25 ∼ 3.21 (m, 2H), 2.57 ∼ 2.49 (m, 2H), 2.40 ∼ 2.33 (m, 2H), 2.11 ∼ 2.02 (m, 2H), 1.84 ∼ 1.80 (m, 2H), 1.70 ∼ 1.61 (m, 3H), 1.37 ∼ 1.24 (m, 22H), 0.87 (t, *J* = 6.8 Hz, 3H)).

### [Example 7]

### Preparation of donepezil ether myristate hydrochloride (DEM HCl) [2-((1-benzylpiperidin-4-yl)methyl)-5,6-dimethoxy-1H-inden-3-yl 2-(dodecyloxy)acetate hydrochloride]

Donepezil ether myristate hydrochloride (DEM HCl) was obtained in a similar manner to Example 6 using the donepezil ether pentadecanoate (DEM) synthesized in Example 2 above.

(¹H NMR (CDCl₃, 400 MHz) δ 12.44 (br, 1H), 7.68 ∼ 7.58 (m, 2H), 7.43∼ 7.39 (m, 3H), 6.98 ∼ 6.93 (m, 1H), 6.57∼6.56 (m, 1H), 4.40 - 4.37 (m, 2H), 4.17 ~4.09 (m, 2H), 3.87 ∼3.85 (m, 6H), 3.63 (t, *J* = 6.7 Hz, 2H), 3.43 ∼ 3.40 (m, 2H), 3.24 ∼ 3.21 (m, 2H), 2.58 ∼ 2.50 (m, 2H), 2.40 ∼ 2.33 (m, 2H) , 2.12 ∼ 2.01 (m, 2H), 1.84 ∼ 1.80 (m, 2H), 1.70 ∼ 1.60 (m, 3H), 1.39 ∼ 1.24 (m, 18H), 0.86 (t, *J* = 6.8 Hz, 3H)).

### [Example 8]

### Preparation of donepezil ether pentadecanoate hydrochloride (DEPD HCl) [2-((1-benzylpiperidin-4-yl)methyl)-5,6-dimethoxy-1H-inden-3-yl 2-(tridecyloxy)acetate hydrochloride]

Donepezil ether pentadecanoate hydrochloride (DEPD HCl) was obtained in a similar manner to Example 6 using the donepezil ether pentadecanoate (DEPD) synthesized in Example 3 above.

(¹H NMR (CDCl₃, 400 MHz) δ 12.39 (br, 1H), 7.67 ∼ 7.57 (m, 2H), 7.42 ∼ 7.38 (m, 3H), 6.97 ∼ 6.92 (m, 1H), 6.57∼6.55 (m, 1H), 4.39 ∼ 4.37 (m, 2H), 4.16 ~ 4.08 (m, 2H), 3.89 ∼3.84 (m, 6H), 3.62 (t, *J* = 6.7 Hz, 2H), 3.42 ∼ 3.39 (m, 2H), 3.24 ∼ 3.20 (m, 2H), 2.58 ∼ 2.50 (m, 2H), 2.40 ∼ 2.32 (m, 2H) , 2.11 ∼ 2.00 (m, 2H), 1.82 ∼ 1.79 (m, 2H), 1.69 ∼ 1.59 (m, 3H), 1.38 ∼ 1.23 (m, 20H), 0.86 (t, *J* = 6.8 Hz, 3H)).

### [Example 9]

### Preparation of donepezil ether heptadecanoate (DEHD HCl) [2-((1-benzylpiperidin-4-yl)methyl)-5,6-dimethoxy-1H-inden-3-yl 2-(pentadecyloxy)acetate hydrochloride]

Donepezil ether heptadecanoate hydrochloride (DEHD HCl) was obtained in a similar manner to Example 6 using the donepezil ether heptadecanoate (DEHD) synthesized in Example 4 above.

(¹H NMR (CDCl₃, 400 MHz) δ 12.44 (br, 1H), 7.68 ~ 7.57 (m, 2H), 7.43 ∼ 7.39 (m, 3H), 6.98 ∼ 6.93 (m, 1H), 6.57 ∼ 6.56 (m, 1H), 4.40 ∼ 4.37 (m, 2H), 4.19 ∼ 4.09 (m, 2H), 3.87 ∼ 3.85 (m, 6H), 3.63 (t, *J* = 6.7 Hz, 2H), 3.43 - 3.40 (m, 2H), 3.24 ∼ 3.21 (m, 2H), 2.58 ∼ 2.50 (m, 2H), 2.40 ∼ 2.33 (m, 2H), 2.12 ∼ 2.01 (m, 2H), 1.84 ∼ 1.80 (m, 2H), 1.70 ∼ 1.58 (m, 3H), 1.39 ∼ 1.24 (m, 24H), 0.87 (t, *J* = 6.8 Hz, 3H)).

### [Example 10]

### Preparation of donepezil ether stearate hydrochloride (DES HCl) [2-((1-benzylpiperidin-4-yl)methyl)-5,6-dimethoxy-1H-inden-3-yl 2-(hexadecyloxy)acetate hydrochloride]

Donepezil ether stearate hydrochloride (DES HCl) was obtained in a similar manner to Example 6 using the donepezil ether stearate (DES) synthesized in Example 5 above.

(¹H NMR (CDCl₃, 400 MHz) δ 12.41 (br, 1H), 7.67 ∼ 7.57 (m, 2H), 7.42 ∼ 7.38 (m, 3H), 6.97 - 6.93 (m, 1H), 6.57 ∼ 6.56 (m, 1H), 4.40 ∼ 4.37 (m, 2H), 4.17 - 4.09 (m, 2H), 3.87 ∼ 3.84 (m, 6H), 3.63 (t, *J* = 6.7 Hz, 2H), 3.42 ∼ 3.39 (m, 2H), 3.24 ∼ 3.21 (m, 2H), 2.58 ∼ 2.50 (m, 2H), 2.40 ∼ 2.33 (m, 2H), 2.11 ∼ 2.01 (m, 2H), 1.83 ∼ 1.80 (m, 2H), 1.69 ∼ 1.59 (m, 3H), 1.38 ∼ 1.23 (m, 26H), 0.86 (t, *J* = 6.8 Hz, 3H)).

### [Example 11]

### Preparation of donepezil compositions

Solution phase compositions were prepared using donepezil (D) and donepezil ether palmitate hydrochloride (DEP HCl).

The main ingredients (D and DEP HCl), castor oil, and benzyl benzoate were mixed according to the compositions and amounts of Table 1 below, and stirred at room temperature for 0.5 ~ 3 hours to prepare the solution phase compositions.

**[Table 1]**

| (Unit: mg) | D (donepezil) | DEP HCl |
|---|---|---|
| Main ingredient | 137 | 242 |
| Castor oil | 1,200 | 750 |
| Benzyl benzoate | 1,800 | 500 |

| | | |
|---|---|---|
| * corresponds to an equivalent dose as donepezil | | |

### [Experimental Example 1]

### Raw Material Accelerated Stability Comparison Test of DEP and DEP HCl

Experiments were conducted to evaluate the raw material accelerated stability over time of donepezil ether palmitate (DEP) and donepezil ether palmitate hydrochloride (DEP HCl).

Specifically, in the experiments, each compound was stored in a light-shielding seal under accelerated conditions (40 °C, RH 75%), and the chemical stability thereof was analyzed by HPLC (purity and increased amount of related substances). Results thereof are shown in Table 2 below.

**[Table 2]**

| Samp le | Initial | | Month 1 | | Month 3 | | Month 6 | |
|---|---|---|---|---|---|---|---|---|
| | Purity | PT impurity | Purity | PT impurity | Purity | PT impurity | Purity | PT impurity |
| DEP | 99.22% | 0.02% | 99.05% | 0.04% | 98.65% | 0.08% | 96.75% | 0.13% |
| DEP HCl | 99.75% | Not detected | 99.74% | Not detected | 99.74% | Not detected | 99.74 % | Not detected |

In the case of DEP, PT impurity increased to 0.13%, and the purity decreased by about 2.47%, based on the 6th month of acceleration despite being stored in a light-shielding seal. On the other hand, even at the 6th month, DEP HCl did not have PT impurity and showed no significant difference from the initial purity, which was confirmed to have excellent stability.

Accordingly, it was confirmed that DEP HCl according to the present disclosure had excellent raw material storage stability under accelerated conditions (40 °C, RH 75%) compared to DEP.

### [Experimental Example 2]

### Raw Material Harsh Stability Comparison Test of DEP and DEP HCl

Experiments were conducted to evaluate the raw material harsh stability over time of donepezil ether palmitate (DEP) and donepezil ether palmitate hydrochloride (DEP HCl).

Specifically, in the experiments, each compound was stored under a condition of 60 °C, and the chemical stability thereof was analyzed by HPLC (purity and increased amount of related substances). Results thereof are shown in Table 3 below.

**[Table 3]**

| Sample | Initial | | Day 1 | | Day 3 | | Week 2 | |
|---|---|---|---|---|---|---|---|---|
| | Purity | PT impurity | Purity | PT impurity | Purity | PT impurity | Purity | PT impurity |
| DEP | 99.76% | Not detected | 95.44% | 1.61% | 92.05% | 3.33% | 81.16% | 4.39% |
| DEP HCl | 99.73% | Not detected | 99.69% | Not detected | 99.59% | Not detected | 99.34% | Not detected |

In the case of DEP, PT impurity continued to increase, which increased to 4.39% based on the 2nd week, and the purity decreased by about 18.60% due to the increase in many other related substances. On the other hand, it was confirmed that the DEP HCl of the present disclosure did not produce PT impurity at all and had a non-significant change in overall purity.

Therefore, it could be appreciated that DEP HCl according to the present disclosure had excellent raw material storage stability even under harsh conditions at a high temperature of 60 °C compared to DEP.

### [Experimental Example 3]

### Formulation Stability Comparison Test of DEP and DEP HCl

Experiments were conducted to evaluate the formulation stability over time of donepezil ether palmitate (DEP) and donepezil ether palmitate hydrochloride (DEP HCl).

Specifically, each of DEP and DEP HCl was mixed with benzyl benzoate and castor oil to prepare a suspension, and then stored at 60 °C and 80 °C. The chemical stability was analyzed by HPLC (purity and increased amount of related substances), and results thereof are shown in Table 4.

**[Table 4]**

| Sample | Initial | | Day 1 at 80 °C | | Week 1 at 60 °C | |
|---|---|---|---|---|---|---|
| | Purity | PT impurity | Purity | PT impurity | Purity | PT impurity |
| DEP | 99.79% | Not detected | 97.96% | 0.62% | 97.54% | 0.82% |
| DEP HCl | 99.78% | Not detected | 98.93% | Not detected | 98.12% | Not detected |

In the case of DEP, PT impurity increased more than the standard in both 60 °C and 80 °C conditions. In contrast, DEP HCl of the present disclosure did not produce PT impurity under each condition, which was confirmed to have significantly excellent stability compared to DEP.

Therefore, it could be appreciated that DEP HCl according to the present disclosure is a compound capable of suppressing the generation of related substances to maintain high purity for a long period of time and having excellent stability.

### [Experimental Example 4]

### Physical Property Comparison Test of DEP and DEP HCl

### - Differential Scanning Calorimetry (DSC) analysis

Physical properties of donepezil ether palmitate (DEP) and donepezil ether palmitate hydrochloride (DEP HCl) were compared through differential scanning calorimetry analysis. Analysis was performed using a differential scanning calorimeter (Mettler Toledo DSC 823) under nitrogen at a scan rate of 10 °C per minute, and results thereof are shown in Table 5 below.

**[Table 5]**

| Sample | Onset | Peak |
|---|---|---|
| DEP | 55.00 °C | 57.40 °C |
| DEP HCl | 122.92 °C | 125.33 °C |

### - Measurement of melting point

Physical properties of donepezil ether palmitate (DEP) and donepezil ether palmitate hydrochloride (DEP HCl) were compared through a melting point analyzer (MPA100). At this time, the range was set of 25 °C ~ 200 °C, the temperature was raised at 5 °C per minute, and measured when each sample was completely melted. Results thereof are shown in Table 6 below.

**[Table 6]**

| Sample | Melting point |
|---|---|
| DEP | 55.1 °C |
| DEP HCl | 121.9 °C |

As a result of the above analysis, DEP was easily dissolved at a low temperature with a melting point of about 55 °C. This low melting point is highly likely to change the properties or purity of raw materials even when exposed to heat for a short period of time. In contrast, it was confirmed that the melting point of DEP HCl was about 122 °C, which was more than twice as high, and thus DEP HCl was not easily dissolved.

Therefore, it was confirmed that DEP HCl according to the present disclosure had more than two times higher thermal stability compared to DEP and had excellent storage stability when storing raw materials.

### [Experimental Example 5]

### Comparison of Pharmacokinetic Evaluation

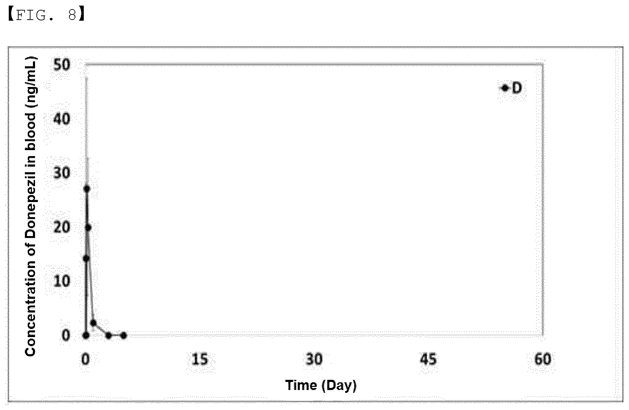

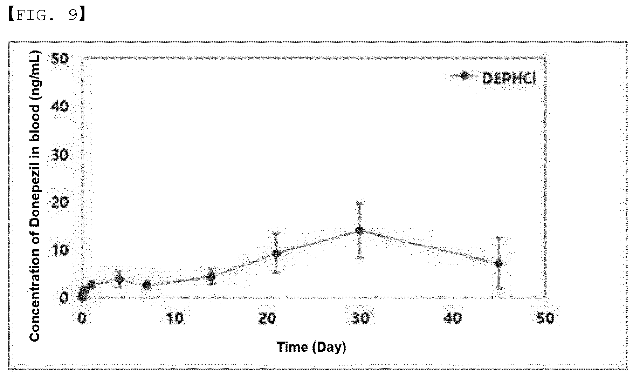

Four male SD rats with an average weight of 300 g were intramuscularly injected with a dose of 4 mg/kg of donepezil in the donepezil (D) formulation and 40 mg/kg of donepezil in the donepezil ether palmitate hydrochloride (DEP HCl) formulation, and the concentration of donepezil in plasma samples of SD rats was analyzed using LC-MS/MS. Results thereof are shown in Table 7, FIG. 8 and FIG. 9 below.

**[Table 7]**

| | D (Donepezil) | DEP HCl |
|---|---|---|
| Cmax (ng/mL) | 27.5 | 13.96 |
| AUClast (ngh/mL) | 367.6 | 6551.14 |

As shown in Table 7 and FIG. 8, it was confirmed that when the donepezil composition was administered, the drug was rapidly released immediately after administration, and the Cmax was as high as 27.5 ng·h/mL, indicating a high possibility of causing side effects or toxicity. Further, the release of the drug was terminated within 3 days, confirming that the donepezil composition was not suitable as a sustained-release composition.

On the other hand, as shown in Table 7 and FIG. 9, it was confirmed that when the composition of donepezil ether palmitate hydrochloride was administered, despite the administration of the amount of donepezil equivalent to about 10 times, Cmax was as low as 13.96 ng·h/mL without rapid release of drug after administration, and a difference between Cmax and maintenance concentration was small. Further, unlike the case where the donepezil composition was administered, it was confirmed that the donepezil ether palmitate hydrochloride composition maintained an effective blood concentration for a period of 8 weeks or longer even with a single administration.

As a result, it may be appreciated that donepezil ether palmitate hydrochloride exhibits a better release pattern than that of donepezil. It may be confirmed that when donepezil ether palmitate hydrochloride is administered into the body, it is possible to be maintained in a sustained-release form for a long period of time without rapid release of the drug after administration, thereby having excellent properties such as minimizing the risk of side effects including toxic reactions while maintaining an effective therapeutic drug concentration for a long time even with a single administration.

## Claims

1. An acid addition salt of a compound represented by the following Chemical Formula 3: 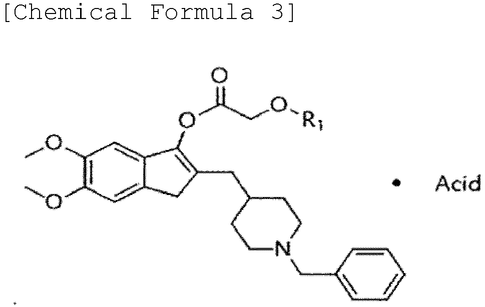
in Chemical Formula above, R₁ is C₁₂-C₁₆ alkyl, and
the acid is hydrochloric acid, sulfuric acid, phosphoric acid, citric acid, acetic acid, formic acid, fumaric acid, benzoic acid, camphorsulfonic acid, cinnamic acid, maleic acid, malic acid, malonic acid, oleic acid, orotic acid, benzenesulfonic acid, tartaric acid, napadisilic acid, tosylic acid, mesylic acid, or succinic acid.

2. 2-((1-Benzylpiperidin-4-yl)methyl)-5,6-dimethoxy-1H-inden-3-yl 2-(tetradecyloxy)acetate hydrochloride represented by the following Chemical Formula 4:

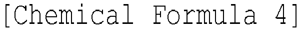

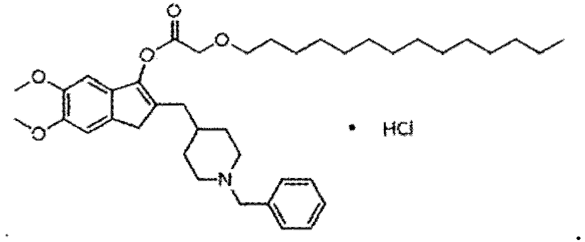

3. A sustained-release pharmaceutical composition comprising a compound represented by the following Chemical Formula 3: 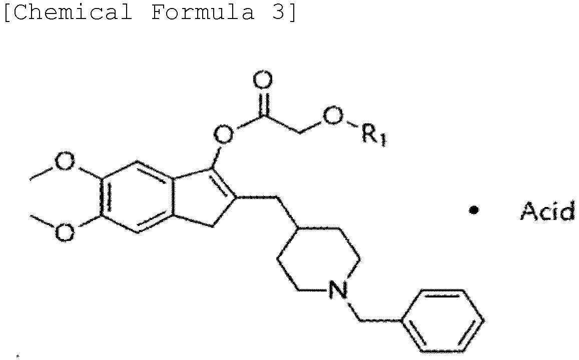
in Chemical Formula above, R₁ is C₁₂-C₁₆ alkyl, and
the acid is hydrochloric acid, sulfuric acid, phosphoric acid, citric acid, acetic acid, formic acid, fumaric acid, benzoic acid, camphorsulfonic acid, cinnamic acid, maleic acid, malic acid, malonic acid, oleic acid, orotic acid, benzenesulfonic acid, tartaric acid, napadisilic acid, tosylic acid, mesylic acid, or succinic acid.

4. A sustained-release pharmaceutical composition comprising 2-((1-benzylpiperidin-4-yl)methyl)-5,6-dimethoxy-1H-inden-3-yl 2-(tetradecyloxy)acetate hydrochloride represented by the following Chemical Formula 4:

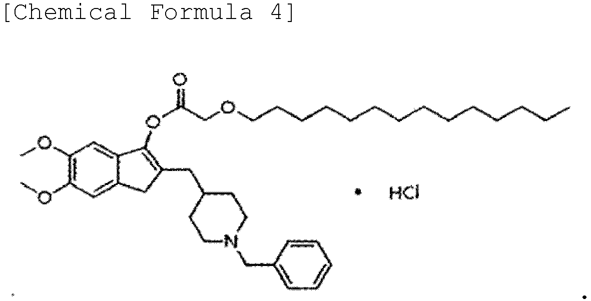

5. The sustained-release pharmaceutical composition of claim 3 or 4, wherein the composition is for preventing or treating dementia.

6. A method for preparing an acid addition salt of 2-((1-benzylpiperidin-4-yl)methyl)-5,6-dimethoxy-1*H*-inden-3-yl 2-(tetradecyloxy)acetate, the method comprising reacting 2-((1-benzylpiperidin-4-yles)methyl)-5,6-dimethoxy-1*H*-inden-3-yl 2-(tetradecyloxy)acetate and acid.

7. The method for preparing an acid addition salt of 2-((1-benzylpiperidin-4-yl)methyl)-5,6-dimethoxy-1*H*-inden-3-yl 2-(tetradecyloxy)acetate of claim 6, which comprises
(1) preparing a solution by adding and dissolving a solvent into 2-((1-benzylpiperidin-4-yl)methyl)-5,6-dimethoxy-1*H*-inden-3-yl 2-(tetradecyloxy)acetate;
(2) forming a salt by adding an acid to the solution prepared in step (1); and
(3) obtaining a solid by stirring the salt prepared in step (2).

8. The method of claim 6 or 7, wherein the acid is hydrochloric acid, sulfuric acid, phosphoric acid, citric acid, acetic acid, formic acid, fumaric acid, benzoic acid, camphorsulfonic acid, cinnamic acid, maleic acid, malic acid, malonic acid, oleic acid, orotic acid, benzenesulfonic acid, tartaric acid, napadisilic acid, tosylic acid, mesylic acid, or succinic acid.

9. The method of claim 7, wherein the solvent is acetone, methanol, ethanol, propanol, isopropyl alcohol, ethyl acetate, isopropyl ether, acetonitrile, tetrahydrofuran, dichloromethane, dioxane, toluene, hexane, heptane, dimethylformamide, dimethylacetamide or a mixture thereof.
